# EUROPEAN PATENT APPLICATION

(11) **EP 4 653 427 A1**
(43) Date of publication of application: **26.11.2025**
(21) Application number: 24744238.7
(22) Date of filing: 16.01.2024
(51) Int. Cl.: C07D 401/04, A61K 31/4545, A61P 37/00

(54) **CRYSTAL FORM OF ENPATORAN, AND PREPARATION METHOD THEREFOR AND USE THEREOF**

(30) Priority: 18.01.2023 CN 202310067091; 18.01.2023 CN 202310066515; 27.04.2023 CN 202310468996
(71) Applicant: Crystal Pharmaceutical (Suzhou) Co., Ltd., Suzhou, Jiangsu 215123 (CN)
(72) Inventor: ZHANG, Yuxing, Suzhou, Jiangsu 215123 (CN); LIU, Jinliang, Suzhou, Jiangsu 215123 (CN); JIANG, Qian, Suzhou, Jiangsu 215123 (CN); ZHANG, Jing, Suzhou, Jiangsu 215123 (CN); MENG, Liping, Suzhou, Jiangsu 215123 (CN)
(74) Representative: dompatent
(86) International application number: PCT/CN2024/072498
(87) International publication number: WO 2024/153063

(57) **Abstract**

The present disclosure relates to novel crystalline forms of Enpatoran (hereinafter referred to as "Compound I") and preparation methods thereof, pharmaceutical compositions containing the crystalline forms, and uses of the crystalline forms for preparing TLR7/8 inhibitor drugs and drugs for treating cutaneous lupus erythematosus and systemic lupus erythematosus.

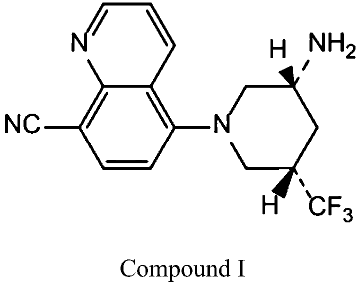

## Description

### TECHNICAL FIELD

The present disclosure pertains to the field of chemical crystallography, particularly relates to novel crystalline forms of Enpatoran, preparation method and use thereof.

### BACKGROUND

Toll-like receptors (TLRs) are a group of pattern recognition receptors in the innate immune system that can recognize conserved microbial structure RNAs such as lipopolysaccharides, flagellin, and viruses. TLR7 and TLR8 recognize microbial single-stranded RNA, purine analogs, and imidazoquinolines, triggering the production of inflammatory cytokines and immune responses to protect the host. Aberrant activation of TLR7/8 is potentially pathogenic and is associated with the progression of autoimmune diseases such as lupus. Lupus is an autoimmune disease that can cause joint pain, fever, skin rashes and organ damage. There are several different types of lupus. Systemic lupus erythematosus (SLE) is the most common, 70% of people with lupus have it. Other types of lupus include cutaneous lupus erythematosus (CLE), drug-induced lupus and neonatal lupus. The global SLE newly diagnosed population was estimated to be 0.40 million people annually. There remains an urgent need for the development of TLR inhibitors for the treatment of lupus.

Enpatoran is a Toll-like receptor 7/8 (TLR7/8) inhibitor developed by Merck that blocks the activation of TLR7 and TLR8. It has shown positive results in clinical studies for the treatment of CLE and SLE. The chemical name of Enpatoran is 5-((3R,5S)-3-amino-5-trifluoromethylpiperidin-1-yl)quinoline-8-carbonitrile (referred to as Compound I hereinafter). The structure of Compound I is disclosed in WO2017106607A1. However no solid form of Compound I is described therein. Moreover, the product obtained by Example 31 in WO2017106607A1 is an amorphous form of Compound I. The structure of Compound I is shown as follows:

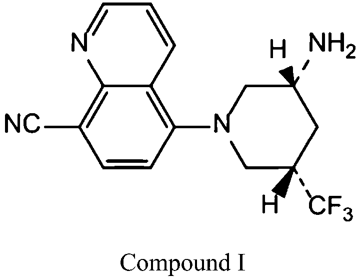

Polymorphism is a common phenomenon in the development of small molecule drugs and represents a key factor affecting drug quality. A crystalline form is a solid material whose constituents are arranged in a highly ordered microscopic structure, forming a crystal lattice that extends in all directions. Polymorphism refers to the phenomenon that a compound exists in more than one crystalline form. Compounds may exist in one or more crystalline forms, but their existence and characteristics cannot be predicted with any certainty.

Different solid forms of an active pharmaceutical ingredient (API) possess distinct physicochemical properties, including chemical stability, thermal stability, solubility, and hygroscopicity. These differences may lead to variations in the dissolution and absorption profiles of the drug in vivo, which can, to some extent, affect its clinical efficacy. In addition, different solid forms of an API may exhibit varying manufacturability characteristics, such as yield, purification behavior, filtration properties, drying efficiency, milling performance, and pressure-related stability during tableting. These differences may influence the processing and handling of the API during drug manufacturing. Therefore, different solid forms of an API may have distinct attributes, offering opportunities to improve the overall performance of the pharmaceutical product.

In the search for new solid forms capable of improving drug performance, the inventors of the present disclosure surprisingly discovered crystalline forms of Compound I, which have advantages in at least one aspect of solubility, hygroscopicity, purification effect, stability, adhesiveness, compressibility, flowability, in vitro and in vivo dissolution, and bioavailability, etc. In particular, the crystalline forms of the Compound I of the present disclosure have advantages such as good physical and chemical stability, low hygroscopicity, high purity due to effective purification, and good stability in pharmaceutical processing, making it more suitable for drug formulation development and is of great significance for the development of drugs containing Compound I.

### SUMMARY

The present disclosure is to provide crystalline solids of Compound I, preparation method, use and pharmaceutical compositions comprising the crystalline solids.

According to the objective of the present disclosure, crystalline solids of Compound I are provided.

According to the objective of the present disclosure, an anhydrate of Compound I is provided.

According to the objective of the present disclosure, a hydrate of Compound I is provided.

According to the objective of the present disclosure, crystalline form CSI of Compound I is provided (hereinafter referred to as Form CSI).

In one aspect provided herein, the X-ray powder diffraction pattern of Form CSI comprises one or two or three characteristic peaks at 2theta values of 8.8°±0.2°, 13.5°±0.2° and 11.4°±0.2° using CuKα radiation. Preferably, the X-ray powder diffraction pattern of Form CSI comprises characteristic peaks at 2theta values of 8.8°±0.2°, 13.5°±0.2° and 11.4°±0.2° using CuKα radiation.

Furthermore, the X-ray powder diffraction pattern of Form CSI comprises one or two or three characteristic peaks at 2theta values of 9.8°±0.2°, 18.5°±0.2° and 21.9°±0.2° using CuKα radiation. Preferably, the X-ray powder diffraction pattern of Form CSI comprises characteristic peaks at 2theta values of 9.8°±0.2°, 18.5°±0.2° and 21.9°±0.2° using CuKα radiation.

Furthermore, the X-ray powder diffraction pattern of Form CSI comprises one or two characteristic peaks at 2theta values of 14.2°±0.2° and 23.5°±0.2° using CuKα radiation. Preferably, the X-ray powder diffraction pattern of Form CSI comprises characteristic peaks at 2theta values of 14.2°±0.2° and 23.5°±0.2° using CuKα radiation.

In another aspect provided herein, the X-ray powder diffraction pattern of Form CSI comprises one or two or three or four or five or six or seven or eight or nine characteristic peaks at 2theta values of 8.8°+0.2°, 13.5°+0.2°, 11.4°+0.2°, 9.8°+0.2°, 18.5°±0.2°, 21.9°±0.2°, 14.2°+0.2°, 23.5°+0.2°, 15.3°+0.2°, 20.6°+0.2° and 25.1°+0.2° using CuKα radiation.

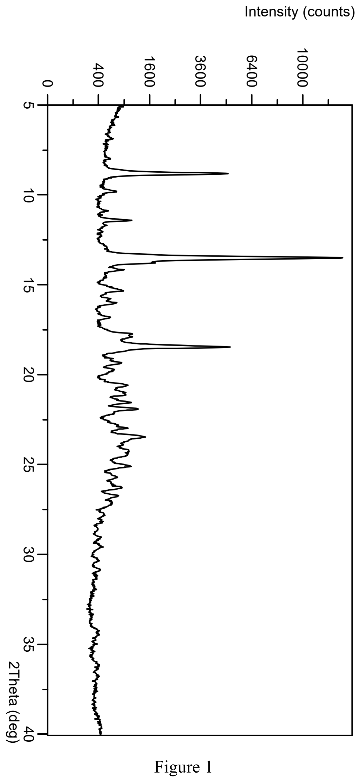

Without any limitation being implied, the X-ray powder diffraction pattern of Form CSI is substantially as depicted in Figure 1 using CuKα radiation.

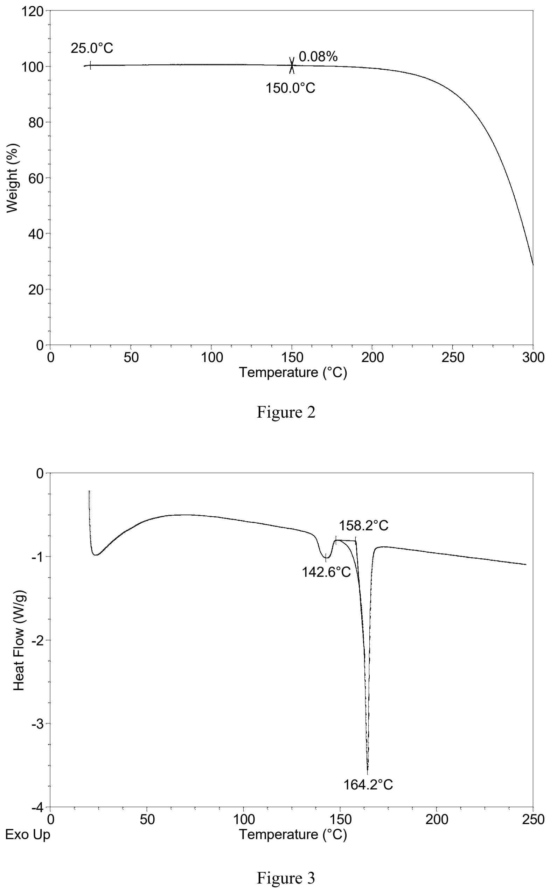

Without any limitation being implied, the TGA curve of Form CSI is substantially as depicted in Figure 2, which shows about 0.08% weight loss when heated to 150 °C.

Without any limitation being implied, the DSC curve of Form CSI shows two endothermic peaks. The first endothermic peak is at around 143 °C (peak temperature), and the second endothermic peak is at around 164 °C (peak temperature).

Without any limitation being implied, the DSC curve of Form CSI is substantially as depicted in Figure 3.

Without any limitation being implied, Form CSI is an anhydrate.

According to the objective of the present disclosure, a process for preparing Form CSI is also provided. The process comprises:
Stirring the solid of Compound I in a mixed solvent of alcohol and water, separating to obtain Form CSI.

Furthermore, said alcohol is preferably methanol. Said volume ratio of alcohol to water in the mixed solvent is preferably 1:5.

Form CSI of the present disclosure exhibits the following unexpected technical effects: (1) Form CSI has lower hygroscopicity.

Under 0%-80%RH condition, the hygroscopic weight gain of Form CSI is 0.36%, whereas the amorphous shows a hygroscopic weight gain of 7.45%. Form CSI with low hygroscopicity is not demanding on the production and storage conditions, which reduces the cost of production, storage and quality control, and has strong economic value.

### (2) Form CSI is suitable for the preparation of pharmaceutical formulations.

Form CSI maintains its crystalline form after grinding in ethanol and remains stable before and after formulation process. While the amorphous undergoes solid-state transformation after grinding in ethanol. Wet granulation is commonly used in the production of solid pharmaceutical formulations, wherein the API is typically mixed with wetting agents such as water or ethanol under mechanical force. Form CSI shows good physical stability in alcoholic solvent and low hygroscopicity, which helps reduce the risk of crystallinity loss and solid-state transformation of the API during the wet granulation process.

### (3) Form CSI has good purification effect.

The purity is significantly increased after converting the raw material into Form CSI. In a specific embodiment, the purity of the raw material used is 97.09%. The purity of Form CSI made from the raw material is 99.00%. The purity is increased by 1.91%. In contrast, preparation of the amorphous from the same raw material led to a 0.23% decrease in purity. Form CSI has good purification effect and is excellent in removing impurities in the crystallization process. APIs with high purity can be obtained through crystallization, which effectively overcome the disadvantages of poor stability, poor efficacy and high toxicity caused by the low purity APIs.

### (4) Form CSI has good stability.

Form CSI shows better humidity stability than the amorphous. After undergoing a humidity cycle of 0%RH-95%RH-0%RH, Form CSI remains its crystalline form, while the amorphous undergoes a solid-state transformation. This indicates that Form CSI has better humidity stability than the amorphous.

Form CSI exhibits better physical and chemical stability than the amorphous. Form CSI maintains its solid-state form and shows no significant change in purity when stored at 40 °C/75%RH for at least 6 months and 60 °C/75%RH for at least 3 months. While amorphous undergoes a solid-state transformation after storing at 60 °C/75%RH for 1 week.

Form CSI formulation shows good stability. When Form CSI is mixed with excipients to form pharmaceutical formulations and stored under the conditions of 25 °C/60%RH, 40 °C/75%RH and 60 °C/75%RH, the solid form and the purity remains unchanged for at least 1 month.

High humidity conditions caused by seasonal changes, regional climate differences and environmental factors can impact the storage, transportation and production of APIs. Form CSI and its formulations show good stability, which helps avoid the impact on drug quality due to solid-state transformation during these processes, ensuring consistent and controllable quality of APIs, minimizing quality change, bioavailability change and toxicity due to crystal transformation.

According to the objective of the present disclosure, crystalline form CSII of Compound I is provided (hereinafter referred to as Form CSII).

In one aspect provided herein, the X-ray powder diffraction pattern of Form CSII comprises one or two or three characteristic peaks at 2theta values of 8.5°±0.2°, 13.1°±0.2° and 10.9°±0.2° using CuKα radiation. Preferably, the X-ray powder diffraction pattern of Form CSII comprises characteristic peaks at 2theta values of 8.5°±0.2°, 13.1°±0.2° and 10.9°±0.2° using CuKα radiation.

Furthermore, the X-ray powder diffraction pattern of Form CSII comprises one or two or three characteristic peaks at 2theta values of 17.6°±0.2°, 18.9°±0.2° and 21.6°±0.2° using CuKα radiation. Preferably, the X-ray powder diffraction pattern of Form CSII comprises characteristic peaks at 2theta values of 17.6°±0.2°, 18.9°±0.2° and 21.6°±0.2° using CuKα radiation.

Furthermore, the X-ray powder diffraction pattern of Form CSII comprises one or two or three characteristic peaks at 2theta values of 18.1°±0.2°, 23.4°±0.2° and 24.5°±0.2° using CuKα radiation. Preferably, the X-ray powder diffraction pattern of Form CSII comprises characteristic peaks at 2theta values of 18.1°±0.2°, 23.4°±0.2° and 24.5°±0.2° using CuKα radiation.

In another aspect provided herein, the X-ray powder diffraction pattern of Form CSII comprises one or two or three or four or five or six or seven or eight or nine characteristic peaks at 2theta values of 8.5°±0.2°, 13.1°±0.2°, 10.9°±0.2°, 17.6°±0.2°, 18.9°±0.2°, 21.6°±0.2°, 18.1°±0.2°, 23.4°±0.2°, 24.5°±0.2°, 21.1°±0.2° and 25.5°±0.2° using CuKα radiation.

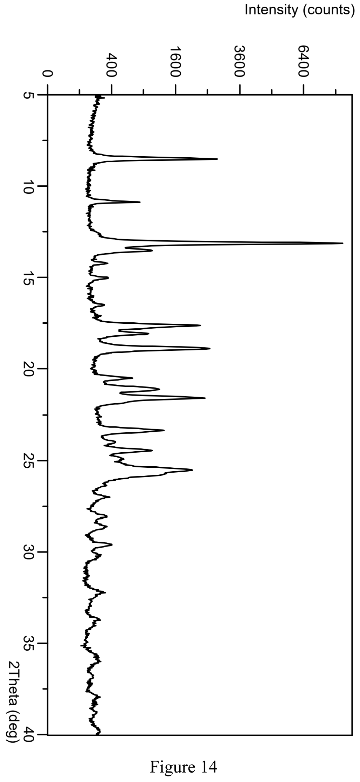

Without any limitation being implied, the X-ray powder diffraction pattern of Form CSII is substantially as depicted in Figure 14 using CuKα radiation.

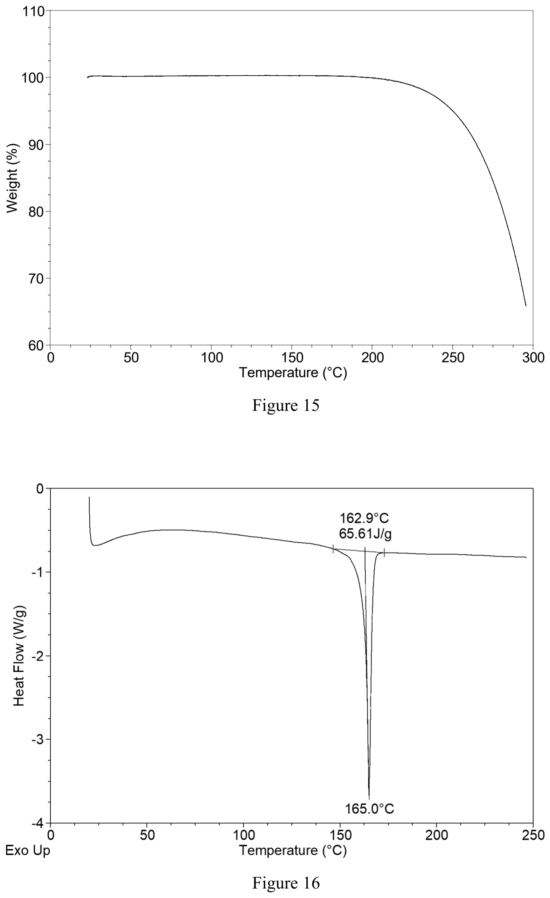

Without any limitation being implied, the TGA curve of Form CSII is substantially as depicted in Figure 15, which shows no significant weight loss when heated to 150 °C.

Without any limitation being implied, the DSC curve of Form CSII shows one endothermic peak. The onset temperature of the endothermic peak is at around 163 °C. Without any limitation being implied, the DSC curve of Form CSII is substantially as depicted in Figure 16.

Without any limitation being implied, Form CSII is an anhydrate.

According to the objective of the present disclosure, a process for preparing Form CSII is also provided. The process comprises:
Heating the solid of Compound I to a temperature of 150 °C-165 °C, then cooling to room temperature to obtain Form CSII.

Form CSII of the present disclosure exhibits the following unexpected technical effects:

### (1) Form CSII has lower hygroscopicity.

Under 0%-80%RH condition, the hygroscopic weight gain of Form CSII is 0.34%, whereas the amorphous shows a hygroscopic weight gain of 7.45%. Form CSII with low hygroscopicity is not demanding on the production and storage conditions, which reduces the cost of production, storage and quality control, and has strong economic value.

### (2) Form CSII is suitable for the preparation of pharmaceutical formulations.

Form CSII maintains its crystalline form after grinding in ethanol and remains stable before and after formulation process. While the amorphous undergoes solid-state transformation after grinding in ethanol. Wet granulation is commonly used in the production of solid pharmaceutical formulations, wherein the API is typically mixed with wetting agents such as water or ethanol under mechanical force. Form CSII shows good physical stability in alcoholic solvent and low hygroscopicity, which helps reduce the risk of crystallinity loss and solid-state transformation of the API during the wet granulation process.

### (3) Form CSII has good purification effect.

The purity is significantly increased after converting the raw material into Form CSII. In a specific embodiment, the purity of the raw material used is 97.09%. The purity of Form CSII made from the raw material is 97.70%. The purity is increased by 0.61%. In contrast, preparation of the amorphous from the same raw material led to a 0.23% decrease in purity. Form CSII has good purification effect. Thus APIs with high purity can be obtained through crystallization, which effectively overcome the disadvantages of poor stability, poor efficacy and high toxicity caused by the low purity APIs.

### (4) Form CSII has good stability.

Form CSII shows better humidity stability than the amorphous. After undergoing a humidity cycle of 0%RH-95%RH-0%RH, Form CSII remains its crystalline form, while amorphous undergoes a solid-state transformation. This indicates that Form CSII has better humidity stability than the amorphous.

Form CSII exhibits better physical and chemical stability than the amorphous. Form CSII maintains its solid-state form and shows no significant change in purity when stored at 40 °C/75%RH for at least 6 months and 60 °C/75%RH for at least 3 months. While amorphous undergoes a solid-state transformation after storing at 60 °C/75%RH for 1 week.

Form CSII formulation shows good stability. When Form CSII is mixed with excipients to form pharmaceutical formulations and stored under conditions of 25 °C/60%RH, 40 °C/75%RH and 60 °C/75%RH, the solid form and the purity remains unchanged for at least 1 month.

High humidity conditions caused by seasonal changes, regional climate differences and environmental factors can impact the storage, transportation and production of APIs. Form CSII and its formulations show good stability, which helps avoid the impact on drug quality due to solid-state transformation during these processes, ensuring consistent and controllable quality of APIs, minimizing quality change, bioavailability change and toxicity due to crystal transformation.

According to the objective of the present disclosure, crystalline form CSIII of Compound I is provided (hereinafter referred to as Form CSIII).

In one aspect provided herein, the X-ray powder diffraction pattern of Form CSIII comprises one or two or three characteristic peaks at 2theta values of 7.1°±0.2°, 10.6°±0.2° and 13.3°±0.2° using CuKα radiation. Preferably, the X-ray powder diffraction pattern of Form CSIII comprises characteristic peaks at 2theta values of 7.1°±0.2°, 10.6°±0.2° and 13.3°±0.2° using CuKα radiation.

Furthermore, the X-ray powder diffraction pattern of Form CSIII comprises one or two or three characteristic peaks at 2theta values of 6.0°±0.2°, 12.2°±0.2° and 17.9°±0.2° using CuKα radiation. Preferably, the X-ray powder diffraction pattern of Form CSIII comprises characteristic peaks at 2theta values of 6.0°±0.2°, 12.2°±0.2° and 17.9°±0.2° using CuKα radiation.

Furthermore, the X-ray powder diffraction pattern of Form CSIII comprises one or two or three characteristic peaks at 2theta values of 20.6°±0.2°, 21.5°±0.2° and 24.8°±0.2° using CuKα radiation. Preferably, the X-ray powder diffraction pattern of Form CSIII comprises characteristic peaks at 2theta values of 20.6°±0.2°, 21.5°±0.2° and 24.8°±0.2° using CuKα radiation.

In another aspect provided herein, the X-ray powder diffraction pattern of Form CSIII comprises one or two or three or four or five or six or seven or eight or nine characteristic peaks at 2theta values of 7.1°±0.2°, 10.6°±0.2°, 13.3°±0.2°, 6.0°±0.2°, 12.2°±0.2°, 17.9°±0.2°, 20.6°±0.2°, 21.5°±0.2°, 24.8°±0.2°, 24.0°±0.2° and 27.9°±0.2° using CuKα radiation.

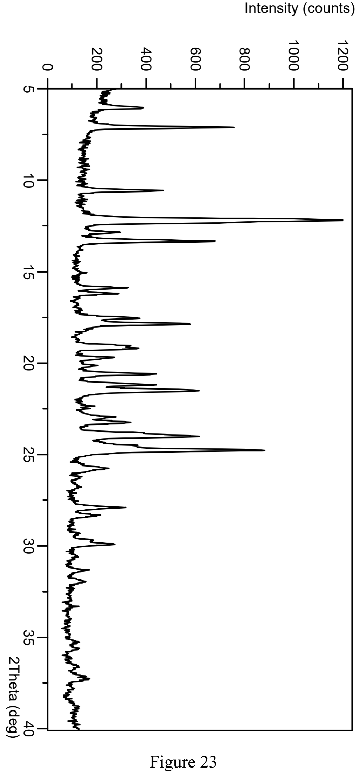

Without any limitation being implied, the X-ray powder diffraction pattern of Form CSIII is substantially as depicted in Figure 23 using CuKα radiation.

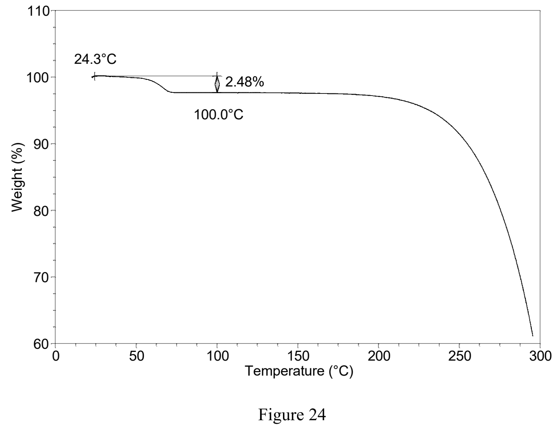

Without any limitation being implied, the TGA curve of Form CSIII is substantially as depicted in Figure 24, which shows about 2.5% weight loss when heated to 100 °C. Without any limitation being implied, Form CSIII is a hydrate.

According to the objective of the present disclosure, a process for preparing Form CSIII is also provided. The process comprises:
Dissolving Compound I in a mixed solvent of nitrile and water, followed by addition of water, stirring to obtain Form CSIII.

Furthermore, said nitrile is preferably acetonitrile. Said volume ratio of acetonitrile to water in the mixed solvent is preferably 3: 1. Said stirring temperature is preferably 5-15 °C, more preferably 5 °C. Said stirring time is preferably 2-24 h, more preferably 4-8 h, further preferably 6 h.

Form CSIII of the present disclosure exhibits the following unexpected technical effects:

### (1) Form CSIII has lower hygroscopicity.

Under 40%-80%RH condition, the hygroscopic weight gain of Form CSIII is 0.83%, whereas the amorphous shows a hygroscopic weight gain of 5.43%. Form CSIII with low hygroscopicity is not demanding on the production and storage conditions, which reduces the cost of production, storage and quality control, and has strong economic value.

### (2) Form CSIII has good purification effect.

The purity is increased after converting the raw material into Form CSIII. In a specific embodiment, the purity of the raw material used is 97.09%. The purity of Form CSIII made from the raw material is 97.51%. The purity is increased by 0.42%. In contrast, preparation of the amorphous from the same raw material led to a 0.23% decrease in purity. Form CSIII has good purification effect. Thus APIs with high purity can be obtained through crystallization, which effectively overcome the disadvantages such as poor stability, poor efficacy and high toxicity caused by the low purity APIs.

According to the objective of the present disclosure, a pharmaceutical composition is provided, said pharmaceutical composition comprises a therapeutically effective amount of crystalline form of Compound I and pharmaceutically acceptable excipients. Furthermore, a pharmaceutical composition is provided, said pharmaceutical composition comprises a therapeutically effective amount of Form CSI, Form CSII or Form CSIII and pharmaceutically acceptable excipients.

According to the objective of the present disclosure, crystalline form of Compound I can be used for preparing drugs antagonizing TLR7/8 receptors.

Furthermore, the present disclosure provides the use of Form CSI, CSII or CSIII in the preparation of TLR7/8 inhibitor drug.

According to the objective of the present disclosure, crystalline form of Compound I can be used for preparing drugs treating cutaneous lupus erythematosus (CLE) and systemic lupus erythematosus (SLE).

Furthermore, the present disclosure provides the use of Form CSI, CSII or CSIII in the preparation of drugs treating CLE and SLE.

### BRIEF DESCRIPTION OF THE DRAWINGS

Figure 1 shows an XRPD pattern of Form CSI
Figure 2 shows a TGA curve of Form CSI
Figure 3 shows a DSC curve of Form CSI
Figure 4 shows an XRPD pattern of amorphous
Figure 5 shows a DVS adsorption plot of Form CSI
Figure 6 shows a DVS adsorption plot of the amorphous
Figure 7 shows an XRPD pattern overlay of Form CSI before and after DVS test (from top to bottom: before DVS, after DVS)
Figure 8 shows an XRPD pattern overlay of the amorphous before and after DVS test (from top to bottom: before DVS, after DVS)
Figure 9 shows an XRPD pattern overlay of Form CSI before and after grinding (from top to bottom: before grinding, after grinding)
Figure 10 shows an XRPD pattern overlay of the amorphous before and after grinding (from top to bottom: before grinding, after grinding)
Figure 11 shows an XRPD pattern overlay of Form CSI before and after storage under different conditions (from top to bottom: initial, 40 °C/75%RH for 6 months, 60 °C/75%RH for 3 months)
Figure 12 shows an XRPD pattern overlay of Form CSI before and after formulation (from top to bottom: blank mixed powder, Form CSI formulation, Form CSI)
Figure 13 shows an XRPD pattern overlay of Form CSI formulation before and after storage under different conditions (from top to bottom: initial, 25 °C/60%RH for 1 month, 40 °C/75%RH for 1 month, 60 °C/75%RH for 1 month)
Figure 14 shows an XRPD pattern of Form CSII
Figure 15 shows a TGA curve of Form CSII
Figure 16 shows a DSC curve of Form CSII
Figure 17 shows a DVS adsorption plot of Form CSII
Figure 18 shows an XRPD pattern overlay of Form CSII before and after DVS test (from top to bottom: before DVS, after DVS)
Figure 19 shows an XRPD pattern overlay of Form CSII before and after grinding (from top to bottom: before grinding, after grinding)
Figure 20 shows an XRPD pattern overlay of Form CSII before and after storage under different conditions (from top to bottom: initial, 40 °C/75%RH for 6 months, 60 °C/75%RH for 3 months)
Figure 21 shows an XRPD pattern overlay of Form CSII before and after formulation (from top to bottom: blank mixed powder, Form CSII formulation, Form CSII)
Figure 22 shows an XRPD pattern overlay of Form CSII formulation before and after storage under different conditions (from top to bottom: initial, 25 °C/60%RH for 1 month, 40 °C/75%RH for 1 month, 60 °C/75%RH for 1 month)
Figure 23 shows an XRPD pattern of Form CSIII
Figure 24 shows a TGA curve of Form CSIII

### DETAILED DESCRIPTION

The present disclosure is further illustrated by the following examples which describe the preparation and use of the crystalline forms of the present disclosure in detail. It is obvious to those skilled in the art that changes in the materials and methods can be accomplished without departing from the scope of the present disclosure.

The abbreviations used in the present disclosure are explained as follows:
RH: Relative humidity
TGA: Thermo Gravimetric Analysis
DSC: Differential Scanning Calorimetry
DVS: Dynamic Vapor Sorption
RRT: Relative Retention Time
¹H NMR: Proton Nuclear Magnetic Resonance
HPLC: High Performance Liquid Chromatography
XRPD: X-ray Powder Diffraction

Instruments and methods used for data collection:
XRPD patterns in the present disclosure were acquired by a Bruker X-ray powder diffractometer. The parameters of the X-ray powder diffraction method of the present disclosure are as follows:
X-Ray source: Cu, Kα
Kα1 (Å): 1.5406; Kα2 (Å): 1.5444
Kα2/Kα1 intensity ratio: 0.50
Voltage: 40 kV
Current: 40 mA
Scan range (2θ): from 3.0 degree to 40.0 degree

TGA data in the present disclosure were acquired by a TA Q500. The parameters of the TGA method of the present disclosure are as follows:
Heating rate: 10 °C/ min
Purge gas: N₂

DSC data in the present disclosure were acquired by a TA Q2000. The parameters of the DSC method of the present disclosure are as follows:
Heating rate: 10 °C/min
Purge gas: N₂

¹H NMR data were collected from a Bruker Avance II DMX 400M HZ NMR spectrometer. 1-5 mg of sample was weighed and dissolved with 0.5 mL of deuterated chloroform to obtain a solution with a concentration of 2-10 mg/mL.

The method for purity detection of the present disclosure is shown in Table 1.

**Table 1**

| | | |
|---|---|---|
| Instrument | Agilent 1260 | |
| Column | Waters XBridge C18, 4.6 mm×150 mm, 5 µm | |
| Mobile phase | A: 0.1% aqueous phosphoric acid solution (pH6.8, triethylamine) | |
| | B: Acetonitrile | |
| Gradient | Time (min) | %B |
| | 0.0 | 10 |
| | 30.0 | 60 |
| | 35.0 | 60 |
| | 36.0 | 10 |
| | 45.0 | 10 |
| Run time | 45.0 min | |
| Flow rate | 1.0 mL/min | |
| Injection volume | 5 µL | |
| Detector wavelength | 248 nm | |
| Column temperature | 40 °C | |
| Sample temperature | Room temperature | |
| Diluent | Methanol | |

In the present disclosure, said "crystalline solid" refers to a solid substance in which molecules are arranged in different patterns and/or conformations within a crystal lattice.

Said "anhydrate" refers to a crystalline solid that does not contain crystalline water or solvent.

Said "hydrate" refers to a crystalline solid that contains crystalline water.

Said "stirring" is accomplished by using a conventional method in the field such as magnetic stirring or mechanical stirring and the stirring speed is 50 to 1800 r/min. Preferably the magnetic stirring speed is 300 to 900 r/min, and mechanical stirring speed is 100 to 300 r/min.

Said "separation" is accomplished by using a conventional method in the field such as centrifugation or filtration. The operation of "centrifugation" is as follows: the sample to be separated is placed into the centrifuge tube, and then centrifuged at a rate of 10000 r/min until the solid all sink to the bottom of the tube.

Said "room temperature" is not a specific temperature, but a temperature range of 10-30 °C.

Said "characteristic peak" refers to a representative diffraction peak used to distinguish crystals, which usually can have a deviation of ±0.2° using CuKα radiation.

In the present disclosure, "crystal" or "crystalline form" refers to the crystal or the crystalline form being identified by the X-ray diffraction pattern shown herein. Those skilled in the art are able to understand that the X-ray powder diffraction pattern depend on the instrument conditions, the sample preparation and the purity of samples. The relative intensity of the diffraction peaks in the X-ray diffraction pattern may also vary with the experimental conditions. Therefore, the order of the diffraction peak intensities cannot be regarded as the sole or decisive factor. In fact, the relative intensity of the diffraction peaks in the X-ray powder diffraction pattern is related to the preferred orientation of the crystals, and the diffraction peak intensities shown herein are illustrative and identical diffraction peak intensities are not required. Thus, it will be understood by those skilled in the art that a crystalline form of the present disclosure is not necessarily to have exactly the same X-ray diffraction pattern of the example shown herein. Any crystalline forms whose X-ray diffraction patterns have the same or similar characteristic peaks should be within the scope of the present disclosure. Those skilled in the art can compare the patterns shown in the present disclosure with that of an unknown crystalline form to identify whether these two groups of patterns reflect the same or different crystalline forms.

In some embodiments, Form CSI, Form CSII or Form CSIII of the present disclosure is pure and substantially free of any other crystalline forms. In the present disclosure, the term "substantially free" when used to describe a novel crystalline form, it means that the content of other crystalline forms in the novel crystalline form is less than 20% (w/w), specifically less than 10% (w/w), more specifically less than 5% (w/w) and furthermore specifically less than 1% (w/w).

In the present disclosure, the term "about" when referring to a measurable value such as weight, time, temperature, and the like, is meant to encompass variations of ± 10%, ± 5%, ± 1%, ± 0.5%, or even ± 0.1% of the specified amount.

Unless otherwise specified, the following examples were conducted at room temperature.

According to the present disclosure, Compound I used as raw materials include, but are not limited to solid (crystalline and amorphous), oil, liquid form or solution. Preferably, Compound I used as the raw material is a solid.

Compound I used in the following examples were prepared by known methods in the prior art, for example, the method disclosed in WO2017106607A1.

### Example 1 Preparation of Form CSI

15.6 mg of Compound I was weighed into a vial, and 0.3 mL of a mixed solvent of methanol and water (1:5, v/v) was added. The mixture was then stirred at room temperature for 5 days. The solid was separated by centrifugation and then vacuum dried at 30 °C for 1.5 hours.

The obtained dried solid was confirmed as Form CSI. The XRPD data are listed in Table 2, and the XRPD pattern is substantially as depicted in Figure 1.

The TGA curve is substantially as depicted in Figure 2, which shows about 0.08% weight loss when heated to 150 °C.

The DSC curve of Form CSI is substantially as depicted in Figure 3, which shows two endothermic peaks. The first endothermic peak was at around 143 °C (peak temperature) and the second endothermic peak was at around 164 °C (peak temperature).

**Table 2**

| 20 (°) | d-spacing (Å) | Intensity (%) |
|---|---|---|
| 8.8 | 10.0 | 34.7 |
| 9.8 | 9.0 | 2.2 |
| 11.4 | 7.8 | 5.3 |
| 13.5 | 6.6 | 100.0 |
| 13.8 | 6.4 | 10.6 |
| 14.2 | 6.3 | 3.6 |
| 15.3 | 5.8 | 3.7 |
| 16.0 | 5.5 | 2.5 |
| 17.8 | 5.0 | 5.3 |
| 18.5 | 4.8 | 36.3 |
| 19.3 | 4.6 | 3.6 |
| 19.7 | 4.5 | 2.3 |
| 20.6 | 4.3 | 4.6 |
| 21.1 | 4.2 | 4.2 |
| 21.5 | 4.1 | 5.4 |
| 21.9 | 4.1 | 6.8 |
| 23.0 | 3.9 | 4.7 |
| 23.5 | 3.8 | 8.4 |
| 24.3 | 3.7 | 4.8 |
| 25.1 | 3.6 | 5.5 |
| 25.7 | 3.5 | 3.0 |
| 26.3 | 3.4 | 3.8 |
| 26.7 | 3.3 | 3.3 |
| 27.2 | 3.3 | 2.2 |

### Example 2 Hygroscopicity and humidity stability of Form CSI

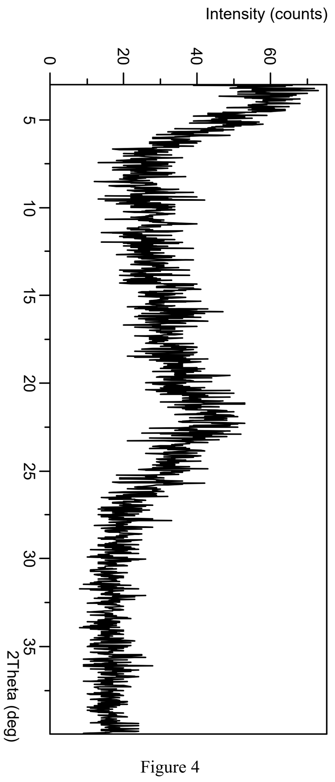

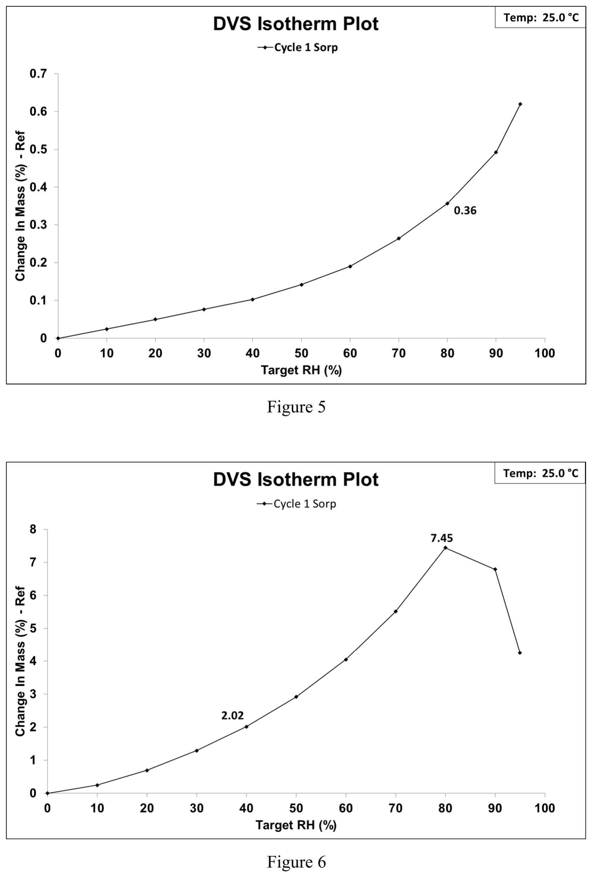

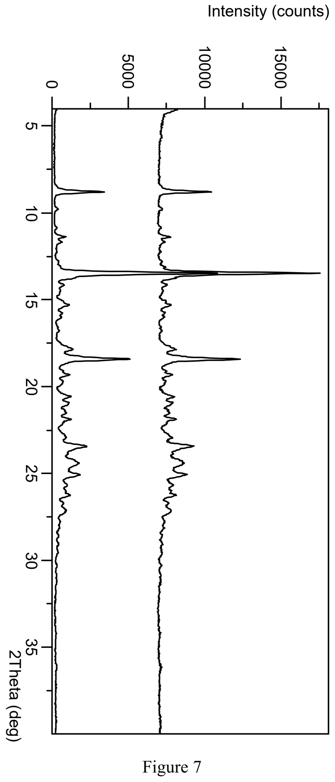

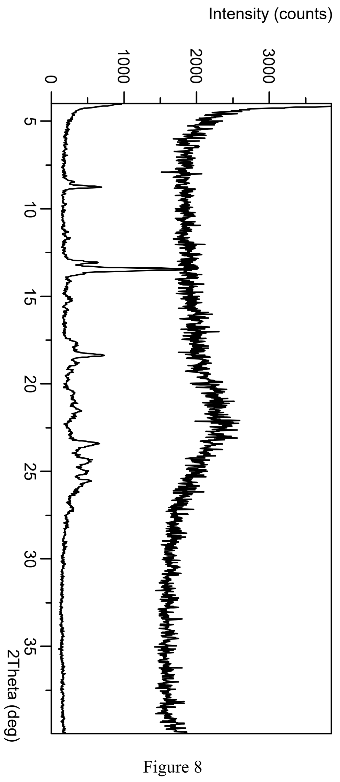

Approximately 10 mg each of Form CSI and the amorphous were weighed and tested for hygroscopicity by DVS instrument. The amorphous was obtained according to Example 31 of WO2017106607A1, and its XRPD pattern is substantially as depicted in Figure 4. The mass at each relative humidity was recorded during a humidity cycle of 0%-95%-0%RH, and the solid form before and after DVS was tested by XRPD. The results are shown in Table 3. The DVS adsorption plots of Form CSI and the amorphous are shown in Figures 5 and 6, respectively. The XRPD overlay before and after DVS test for Form CSI and the amorphous are shown in Figures 7 and 8, respectively.

The results show that the weight gain of Form CSI from 0%RH to 80%RH is 0.36%, and no form change is observed after undergoing a 0%-95%-0%RH humidity cycle. The weight gain of the amorphous from 0%RH to 80%RH is 7.45%, and the solid form changed after undergoing a 0%-95%-0%RH humidity cycle. The results indicate that Form CSI has lower hygroscopicity and better humidity stability than the amorphous.

**Table 3**

| Solid form before DVS test | Weight gain (0%RH to 80%RH) | Solid form after DVS test |
|---|---|---|
| Form CSI | 0.36% | Form CSI |
| The amorphous | 7.45% | Not amorphous |

### Example 3 Purification effect of Form CSI

Form CSI and the amorphous were prepared with the same starting material. The chemical purity of starting material, Form CSI and amorphous was determined by HPLC. The results are listed in Table 4. Compared with the amorphous, Form CSI prepared from the same starting material exhibits higher purity and demonstrates superior purification efficiency for impurities with RRT of 0.30 and 1.57.

**Table 4**

| Solid form | Chemical purity | Purity increment | RRT=0.30 | RRT=1.57 |
|---|---|---|---|---|
| Starting material | 97.09% | - | 0.21% | 2.33% |
| Form CSI | 99.00% | 1.91% | ND | 0.86% |
| The amorphous | 96.86% | -0.23% | 0.22% | 2.64% |

| | | | | |
|---|---|---|---|---|
| ND: Not detected | | | | |

### Example 4 Physical stability of Form CSI under mechanical force

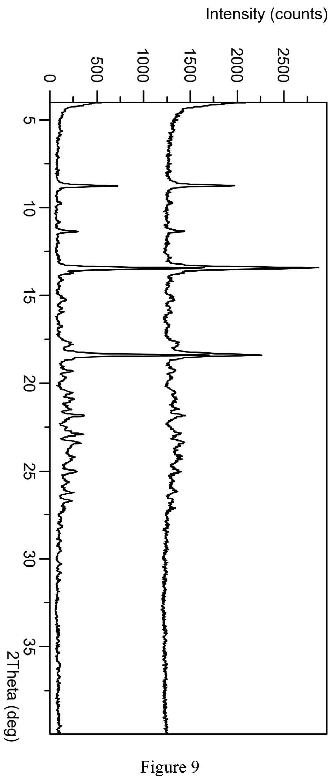

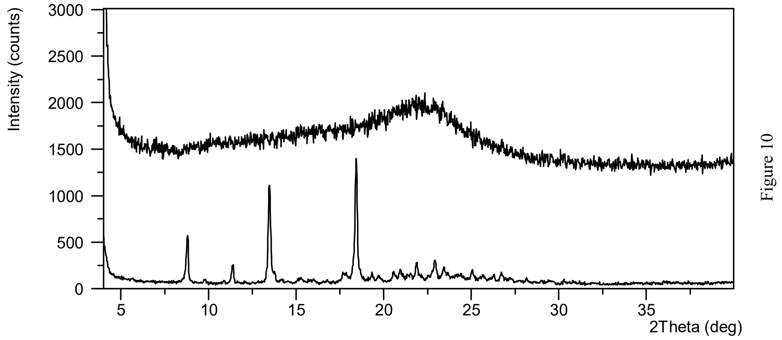

Approximately10 mg of Form CSI and the amorphous were placed in centrifuge tubes, followed by addition of zirconia beads and 5 µL of ethanol. The samples were subjected to ball milling at 500 rpm for 5 minutes. XRPD analyses were performed before and after milling, and the results are shown in Figures 9 and 10. The results indicate that Form CSI remained unchanged after ball milling, whereas amorphous underwent a solid-state transformation. Form CSI has better stability under mechanical force.

### Example 5 Physical and chemical stability of Form CSI

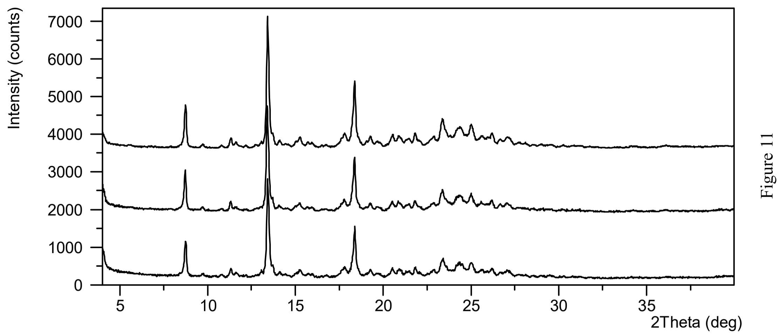

An appropriate amount of Form CSI samples was packaged with desiccant in sealed containers, and then stored under different conditions of 40 °C/75%RH and 60 °C/75%RH. The purity and solid form were analyzed by HPLC and XRPD. The results are shown in Table 5, and the XRPD overlay is substantially as depicted in Figure 11. The results showed that Form CSI remains stable for at least 6 months at 40 °C/75%RH and 3 months at 60 °C/75%RH, indicating Form CSI has good stability under both accelerated and stress conditions. The amorphous undergoes a solid-state transformation after one week at 60 °C/75%RH, suggesting the amorphous has inferior physical stability under stress condition.

**Table 5**

| Initial | Condition | Time | Solid form | Purity (%) |
|---|---|---|---|---|
| Form CSI | Initial | - | Form CSI | 99.11 |
| | 40 °C/75%RH | 6 months | Form CSI | 99.05 |
| | 60 °C/75%RH | 3 months | Form CSI | 99.11 |

### Example 6 Preparation of Form CSI formulation

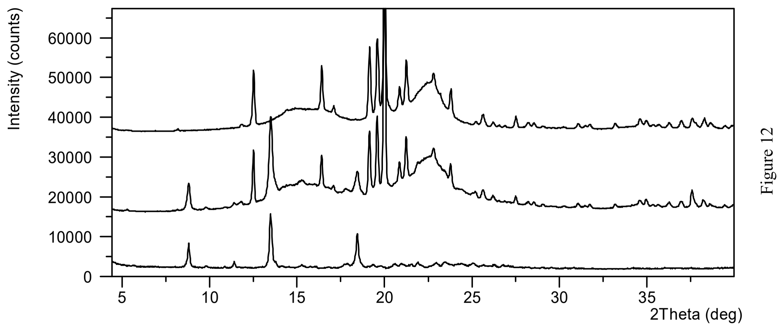

Form CSI formulation and the blank mixed powder formulation were prepared as the formulation compositions shown in Tables 6 and 7 and the formulation process described in Table 8. XRPD analysis was conducted on the blank mixed powder and the Form CSI samples before and after formulation. The results are shown in Figure 12, indicating that Form CSI remained stable throughout the formulation process.

**Table 6**

| No. | Component | mg/unit | % (w/w) |
|---|---|---|---|
| 1 | Compound I | 20 | 20 |
| 2 | Microcrystalline cellulose | 50 | 50 |
| 3 | Lactose | 24 | 24 |
| 4 | Sodium carboxymethyl starch | 5 | 5 |
| 5 | Magnesium stearate | 0.5 | 0.5 |
| 6 | Magnesium stearate | 0.5 | 0.5 |
| Total | | 100 | 100 |

**Table 7**

| No. | Component | mg/unit | % (w/w) |
|---|---|---|---|
| 1 | Microcrystalline cellulose | 62.5 | 62.5 |
| 2 | Lactose | 30 | 30 |
| 3 | Sodium carboxymethyl starch | 6.25 | 6.25 |
| 4 | Magnesium stearate | 1.25 | 1.25 |
| Total | | 100 | 100 |

**Table 8**

| Stage | Procedure |
|---|---|
| Pre-blending | Materials No. 1-5 were weighed according to the formulation composition and transferred into an LDPE bag and manually blended for 2 minutes. |
| Simulated dry granulation | A Φ20 mm round die was installed in an ENERPAC single-punch manual tablet press. The pre-blended powder was compressed into flakes under a pressure of 5 ± 1 kN. The flakes were gently crushed and passed through a 20-mesh sieve. |
| Final blending | The granules were blended with external excipient No.6 for 2 minutes. |
| Tableting | A T 9×4 punch was installed. The final blend (100 ± 1 mg) was compressed into tablets with a pressure of 5 kN. |
| Packaging | One tablet with 2 g of desiccant and 1 g of antioxidant was placed in a 35 cc HDPE bottle and sealed. |

### Example 7 Stability of Form CSI formulation

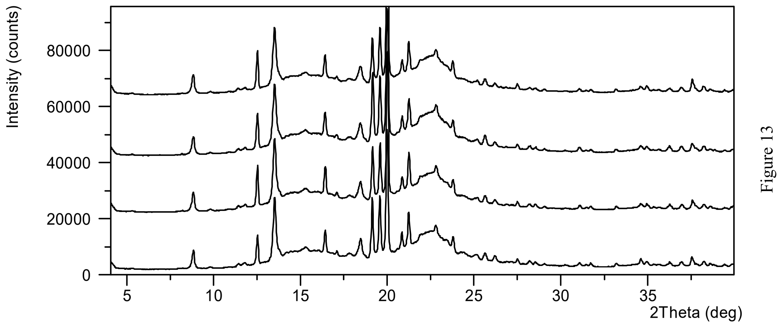

An appropriate amount of Form CSI formulation samples was packaged with desiccant and antioxidant in sealed containers, and stored under conditions of 25 °C/60%RH, 40 °C/75%RH and 60 °C/75%RH. The purity and solid form were determined by HPLC and XRPD. The results are shown in Table 9 and Figure 13, indicating that Form CSI formulation remained stable for at least 1 month under conditions of 25 °C/60%RH, 40 °C/75%RH and 60 °C/75%RH, with no significant changes in purity. It can be seen that Form CSI formulation has good stability under long-term, accelerated and stress conditions.

**Table 9**

| Initial | Condition | Time | Solid form |
|---|---|---|---|
| Form CSI | Initial | - | Form CSI |
| | 25 °C/60%RH | 1 month | Form CSI |
| | 40 °C/75%RH | 1 month | Form CSI |
| | 60 °C/75%RH | 1 month | Form CSI |

### Example 8 Preparation of Form CSII

A solid of Compound I was heated to 155 °C at a rate of 10 °C/min under nitrogen protection, held at 155 °C for 5 minutes and then cooled to room temperature to obtain a dried solid.

The obtained dried solid was detected as Form CSII of the present disclosure. The XRPD data are listed in Table 10, and the XRPD pattern is substantially as depicted in Figure 14.

The TGA curve is substantially as depicted in Figure 15, no significant weight loss was observed up to about 150 °C.

The DSC curve is substantially as depicted in Figure 16, which shows one melting endothermic peak at around 163 °C (onset temperature).

**Table 10**

| 20 (°) | d-spacing (Å) | Intensity (%) |
|---|---|---|
| 8.5 | 10.4 | 31.6 |
| 10.9 | 8.1 | 8.1 |
| 13.1 | 6.7 | 100.0 |
| 13.5 | 6.6 | 10.9 |
| 14.2 | 6.2 | 2.1 |
| 15.0 | 5.9 | 2.4 |
| 16.5 | 5.4 | 1.9 |
| 17.6 | 5.0 | 25.5 |
| 18.1 | 4.9 | 10.1 |
| 18.9 | 4.7 | 29.0 |
| 20.5 | 4.3 | 6.7 |
| 21.1 | 4.2 | 12.7 |
| 21.6 | 4.1 | 27.1 |
| 23.4 | 3.8 | 14.1 |
| 24.0 | 3.7 | 3.6 |
| 24.5 | 3.6 | 11.0 |
| 25.5 | 3.5 | 22.7 |
| 25.8 | 3.5 | 13.2 |
| 27.0 | 3.3 | 2.5 |
| 28.1 | 3.2 | 2.4 |
| 28.6 | 3.1 | 2.1 |
| 29.6 | 3.0 | 3.1 |
| 30.2 | 3.0 | 1.4 |
| 32.2 | 2.8 | 1.6 |
| 35.9 | 2.5 | 1.1 |

### Example 9 Hygroscopicity and humidity stability of Form CSII

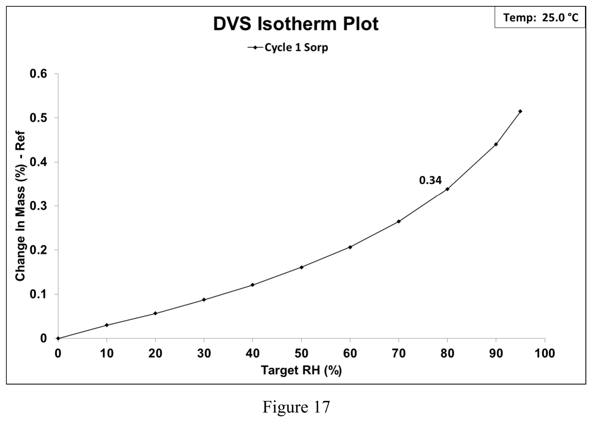

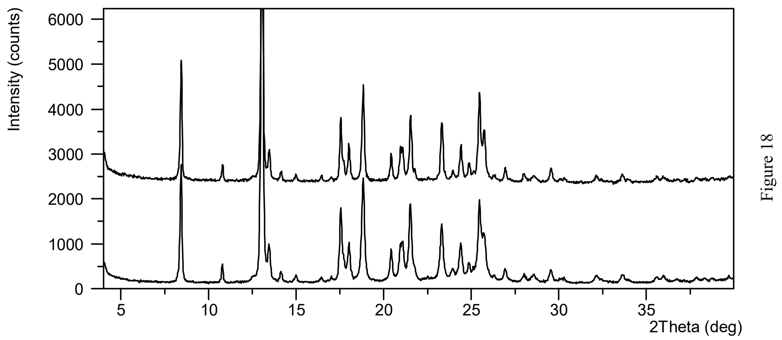

Approximately 10 mg each of Form CSII and the amorphous were weighed and tested for hygroscopicity by DVS instrument. The mass at each relative humidity was recorded during a humidity cycle of 0%-95%-0%RH, and the solid form before and after DVS was tested by XRPD. The results are shown in Table 11. The DVS adsorption plot of Form CSII is shown in Figure 17. The XRPD overlay before and after DVS test for Form CSII is shown in Figure 18.

The results show that the weight gain of Form CSII from 0%RH to 80%RH is 0.34%, and no form change is observed after undergoing a 0%-95%-0%RH humidity cycle. The weight gain of the amorphous from 0%RH to 80%RH is 7.45%, and the solid form changed after undergoing a 0%-95%-0%RH humidity cycle. The results indicate that Form CSII has lower hygroscopicity and better humidity stability than the amorphous.

**Table 11**

| Solid form before DVS test | Weight gain (0%RH to 80%RH) | Solid form after DVS test |
|---|---|---|
| Form CSII | 0.34% | Form CSII |
| Amorphous | 7.45% | Not amorphous |

### Example 10 Purification effect of Form CSII

Form CSII and the amorphous were prepared with the same starting material. The chemical purity of starting material, Form CSII and the amorphous was determined by HPLC. The results are listed in Table 12. Compared with the amorphous, Form CSII prepared from the same starting material exhibits higher purity and demonstrates superior purification efficiency for impurities with RRT of 0.30 and 1.57.

**Table 12**

| Solid form | Chemical purity | Purity increment | RRT=0.30 | RRT=1.57 |
|---|---|---|---|---|
| Starting material | 97.09% | - | 0.21% | 2.33% |
| Form CSII | 97.70% | 0.61% | 0.06% | 2.02% |
| Amorphous | 96.86% | -0.23% | 0.22% | 2.64% |

### Example 11 Physical stability of Form CSII under mechanical force

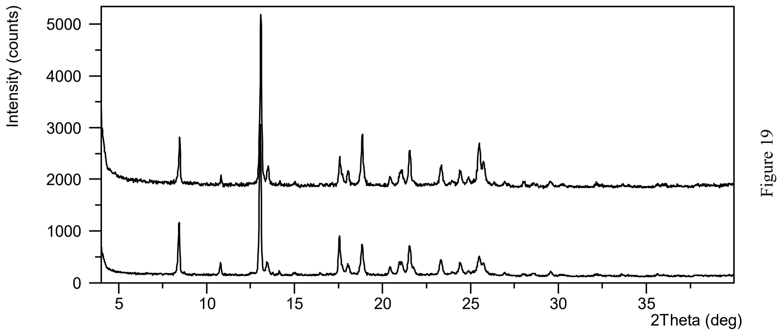

Approximately 10 mg of Form CSII and the amorphous were placed in centrifuge tubes, followed by addition of zirconia beads and 5 µL of ethanol. The samples were subjected to ball milling at 500 rpm for 5 minutes. XRPD analyses were performed before and after milling, and the results are shown in Figures 19 and 10. The results indicate that Form CSII remained unchanged after ball milling, whereas the amorphous underwent a solid-state transformation. Form CSII has better stability under mechanical force than the amorphous.

### Example 12 Physical and chemical stability of Form CSII

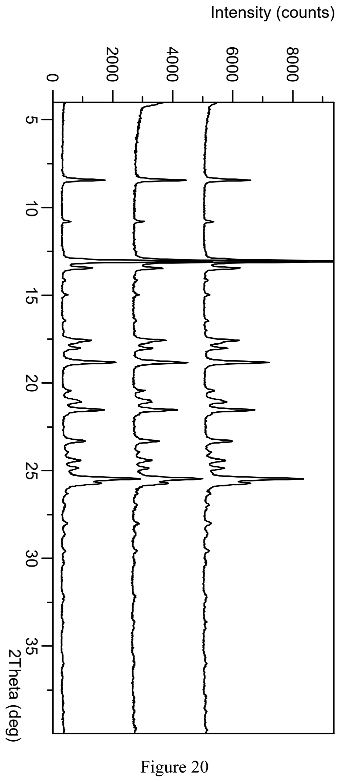

An appropriate amount of Form CSII samples was packaged with desiccant in sealed containers, and then stored under different conditions of 40 °C/75%RH and 60 °C/75%RH. The purity and solid form were analyzed by HPLC and XRPD. The results are shown in Table 13, and the XRPD overlay is substantially as depicted in Figure 20. The results showed that Form CSII remains stable for at least 6 months at 40 °C/75%RH and 3 months at 60 °C/75%RH, indicating Form CSII has good stability under both accelerated and stress conditions. The amorphous undergoes a solid-state transformation after one week at 60 °C/75%RH, suggesting the amorphous has inferior physical stability under stress condition.

**Table 13**

| Initial | Condition | Time | Solid form | Purity (%) |
|---|---|---|---|---|
| Form CSII | Initial | - | Form CSII | 99.02 |
| | 40 °C/75%RH | 6 months | Form CSII | 99.00 |
| | 60 °C/75%RH | 3 months | Form CSII | 99.02 |

### Example 13 Preparation of Form CSII drug product

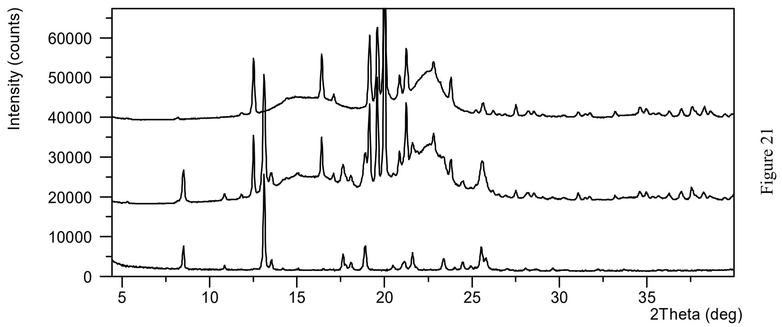

Form CSII formulation and the blank mixed powder formulation were prepared as the formulation compositions shown in Tables 6 and 7 and the formulation process described in Table 8. XRPD analysis was conducted on the blank mixed powder and the Form CSII samples before and after formulation. The results are shown in Figure 21, indicating that Form CSII remained stable throughout the formulation process.

### Example 14 Stability of Form CSII in drug product

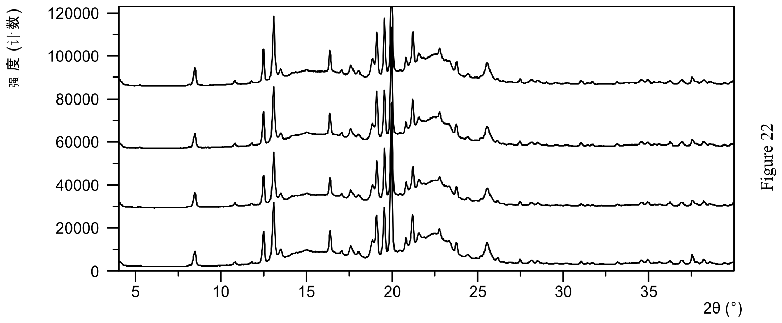

An appropriate amount of Form CSII formulation samples was packaged with desiccant and antioxidant in sealed containers, and stored under conditions of 25 °C/60%RH, 40 °C/75%RH and 60 °C/75%RH. The purity and solid form were determined by HPLC and XRPD. The results are shown in Table 14 and Figure 22, indicating that Form CSII formulation remained stable for at least 1 month under conditions of 25 °C/60%RH, 40 °C/75%RH and 60 °C/75%RH, with no significant changes in purity. It can be seen that Form CSII formulation has good stability under long-term, accelerated and stress conditions.

**Table 14**

| Initial | Condition | Time | Solid form |
|---|---|---|---|
| Form CSII | Initial | - | Form CSII |
| | 25 °C/60%RH | 1 month | Form CSII |
| | 40 °C/75%RH | 1 month | Form CSII |
| | 60 °C/75%RH | 1 month | Form CSII |

### Example 15 Preparation of Form CSIII

10.4 mg of Compound I solid was weighed into a glass vial, and 0.05 mL of a mixed solvent of acetonitrile and water (3:1, v/v) was added. The mixture was heated at 50 °C for about 1.5 h, then transferred to 5 °C and left standing for 1 day. Subsequently, it was transferred to -20 °C and left standing for 3 days. After stirring at -20 °C for 7 days, the mixture was transferred to 5 °C. 1 mL of water was added under stirring and aged for 6 h. The mixture was allowed to stand overnight at 5 °C, followed by centrifugation to isolate the solid. The resulting solid was dried at room temperature overnight.

The obtained dried solid was confirmed as Form CSIII. The XRPD data are listed in Table 15, and the XRPD pattern is substantially as depicted in Figure 23.

**Table 15**

| 20 (°) | d-spacing (Å) | Intensity (%) |
|---|---|---|
| 6.0 | 14.6 | 16.6 |
| 7.1 | 12.4 | 55.0 |
| 10.6 | 8.4 | 32.1 |
| 12.2 | 7.3 | 100.0 |
| 12.9 | 6.9 | 16.5 |
| 13.3 | 6.6 | 54.0 |
| 15.9 | 5.6 | 20.2 |
| 16.2 | 5.5 | 17.1 |
| 17.5 | 5.1 | 24.4 |
| 17.9 | 5.0 | 45.0 |
| 19.2 | 4.6 | 25.1 |
| 19.7 | 4.5 | *15.5* |
| 20.6 | 4.3 | 31.6 |
| 21.2 | 4.2 | 32.2 |
| 21.5 | 4.1 | 48.7 |
| 23.0 | 3.9 | 15.0 |
| 23.2 | 3.8 | 22.4 |
| 24.0 | 3.7 | 49.2 |
| 24.8 | 3.6 | 74.7 |
| 25.8 | 3.5 | 13.2 |
| 27.9 | 3.2 | 21.1 |
| 28.3 | 3.2 | 9.8 |
| 29.9 | 3.0 | 16.4 |
| 31.4 | 2.9 | 5.4 |
| 37.3 | 2.4 | 7.6 |

### Example 16 TGA curve of Form CSIII

The TGA curve of Form CSIII is substantially as depicted in Figure 24, which shows a weight loss of 2.5% when heated to about 150 °C.

### Example 17 Hygroscopicity and humidity stability of Form CSIII

Approximately 10 mg each of Form CSIII and the amorphous were weighed and tested for hygroscopicity by DVS instrument. The mass at each relative humidity was recorded during a humidity cycle of 40%-95%-0%RH. The results show that the weight gain of Form CSIII from 40%RH to 80%RH is 0.83%, whereas that of the amorphous over the same humidity range is 5.43%. The results indicate that Form CSIII has lower hygroscopicity than the amorphous.

### Example 18 Purification effect of Form CSIII

Form CSIII and the amorphous were prepared with the same starting material. The chemical purity of starting material, Form CSIII and the amorphous was determined by HPLC. The results are listed in Table 16. Compared with the amorphous, Form CSIII prepared from the same starting material exhibits higher purity and demonstrates superior purification efficiency for impurities with RRT of 0.30 and 1.57.

**Table 16**

| Solid form | Chemical purity | Purity increment | RRT=0.30 | RRT=1.57 |
|---|---|---|---|---|
| Starting material | 97.09% | - | 0.21% | 2.33% |
| Form CSIII | 97.51% | 0.42% | 0.11% | 2.11% |
| Amorphous | 96.86% | -0.23% | 0.22% | 2.64% |

The examples described above are only for illustrating the technical concepts and features of the present disclosure, and intended to make those skilled in the art being able to understand the present disclosure and thereby implement it, and should not be concluded to limit the protective scope of this disclosure. Any equivalent variations or modifications according to the spirit of the present disclosure should be covered by the protective scope of the present disclosure.

## Claims

1. A crystalline solid of Compound I,

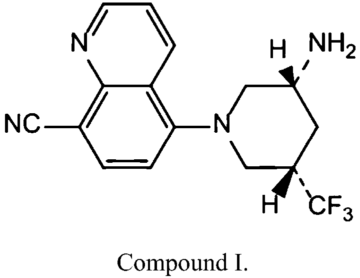

2. The crystalline solid of Compound I according to claim 1, which is an anhydrate.

3. The crystalline solid of Compound I according to claim 1, wherein the X-ray powder diffraction pattern comprises characteristic peaks at 2theta values of 8.8°±0.2°, 13.5±0.2° and 11.4°±0.2° using Cu-Kα radiation.

4. The crystalline solid of Compound I according to claim 3, wherein the X-ray powder diffraction pattern comprises at least one characteristic peak at 2theta values of 9.8°±0.2°, 18.5±0.2° and 21.9°±0.2° using Cu-Kα radiation.

5. The crystalline solid of Compound I according to claim 4, wherein the X-ray powder diffraction pattern comprises at least one characteristic peaks at 2theta values of 14.2°±0.2° and 23.5°±0.2° using Cu-Kα radiation.

6. The crystalline solid of Compound I according to claim 3, wherein the X-ray powder diffraction pattern is substantially as depicted in Figure 1 using Cu-Kα radiation.

7. The crystalline solid of Compound I according to claim 1, wherein the X-ray powder diffraction pattern comprises characteristic peaks at 2theta values of 8.5°±0.2°, 13.1±0.2° and 10.9°±0.2° using Cu-Kα radiation.

8. The crystalline solid of Compound I according to claim 7, wherein the X-ray powder diffraction pattern comprises at least one characteristic peak at 2theta values of 17.6°±0.2°, 18.9°±0.2° and 21.6°±0.2° using Cu-Kα radiation.

9. The crystalline solid of Compound I according to claim 8, wherein the X-ray powder diffraction pattern comprises at least one characteristic peak at 2theta values of 18.1°±0.2°, 23.4°±0.2° and 24.5°±0.2° using Cu-Kα radiation.

10. The crystalline solid of Compound I according to claim 7, wherein the X-ray powder diffraction pattern is substantially as depicted in Figure 14 using Cu-Kα radiation.

11. The crystalline solid of Compound I according to claim 1, which is a hydrate.

12. The crystalline solid of Compound I according to claim 1, wherein the X-ray powder diffraction pattern comprises characteristic peaks at 2theta values of 7.1°±0.2°, 10.6+0.2° and 13.3°±0.2° using Cu-Kα radiation.

13. The crystalline solid of Compound I according to claim 12, wherein the X-ray powder diffraction pattern comprises at least one characteristic peak at 2theta values of 6.0°±0.2°, 12.2°±0.2° and 17.9°±0.2° using Cu-Kα radiation.

14. The crystalline solid of Compound I according to claim 13, wherein the X-ray powder diffraction pattern comprises at least one characteristic peak at 2theta values of 20.6°±0.2°, 21.5°±0.2° and 24.8°±0.2° using Cu-Kα radiation.

15. The crystalline solid of Compound I according to claim 12, wherein the X-ray powder diffraction pattern is substantially as depicted in Figure 23 using Cu-Kα radiation.

16. A pharmaceutical composition, wherein said pharmaceutical composition comprises a therapeutically effective amount of crystalline solid of Compound I according to claim 1, and pharmaceutically acceptable excipients.

17. A method of preparing drugs for antagonizing TLR7/8 receptors, comprising using crystalline solid of Compound I according to claim 1.

18. A method of preparing drugs for treating cutaneous lupus erythematosus (CLE) and systemic lupus erythematosus (SLE), comprising using crystalline solid of Compound I according to claim 1.
